# EUROPEAN PATENT SPECIFICATION

(11) **EP 3 952 872 B1**
(45) Date of publication and mention of the grant of the patent: **13.03.2024**
(21) Application number: 20719737.7
(22) Date of filing: 09.04.2020
(51) Int. Cl.: A61K 31/496, A61K 31/5377, A61K 45/06, A61P 25/00

(54) **CARBAMOYL CYCLOHEXANE DERIVATIVES FOR TREATING AUTISM SPECTRUM DISORDER**
CARBAMOYL-CYCLOHEXAN-DERIVATE ZUR BEHANDLUNG VON AUTISMUSSPEKTRUMSTÖRUNG
DÉRIVÉS DU CARBAMOYL CYCLOHEXANE POUR TRAITER LES TROUBLES DU SPECTRE AUTISTIQUE

(30) Priority: 10.04.2019 HU 1900121
(43) Date of publication of application: 16.02.2022
(73) Proprietor: Richter Gedeon Nyrt., 1103 Budapest (HU)
(72) Inventor: ROMÁN, Viktor, 2030 Érd (HU); ADHAM-PARANGI, Nika, 1103 Budapest (HU); ROGER EARLEY, Willie, 1103 Budapest (HU); PO-JEN YEUNG, Paul, 1103 Budapest (HU)
(74) Representative: Hoffmann Eitle
(86) International application number: PCT/IB2020/053382
(87) International publication number: WO 2020/208564

(56) References cited:
- WO-A1-2005/012266
- WO-A1-2009/020897
- WO-A2-2011/060363
- Wright Jessica: "DSM-5 redefines autism", Spectrum - Autism research News, 21 May 2013 (2013-05-21), pages 1-2, Retrieved from the Internet: URL:https://www.spectrumnews.org/opinion/d sm-5-redefines-autism/
- Anonymous: "Asperger syndrome", Wikipedia, 18 May 2022 (2022-05-18), pages 1-29, Retrieved from the Internet: URL:https://en.wikipedia.org/wiki/Asperger _syndrome [retrieved on 2023-09-19]
- Anonymous: "Pervasive developmental disorder not otherwise specified", Wikipedia, 28 April 2022 (2022-04-28), pages 1-4, Retrieved from the Internet: URL:https://en.wikipedia.org/wiki/Pervasiv e_developmental_disorder_not_otherwise_spe cified [retrieved on 2023-09-19]
- Anonymous: "Childhood disintegrative disorder", Wikipedia, 15 January 2022 (2022-01-15), pages 1-5, Retrieved from the Internet: URL:https://en.wikipedia.org/wiki/Childhoo d_disintegrative_disorder [retrieved on 2023-09-19]

## Description

### FIELD OF THE INVENTION

The present invention relates to trans-N-[4-[2-[4-(2,3-dichlorophenyl)piperazin-1-yl]ethyl]cyclohexyl]-N',N'-dimethylurea (cariprazine), its salts, close analogs, derivatives, pharmaceutical compositions, metabolites and combinations for use in the treatment of symptoms of autism spectrum disorder.

### BACKGROUND OF THE INVENTION

Autism spectrum disorder (ASD) is a complex, very challenging and prevalent neurodevelopmental condition with frequencies as high as 1:34 - 1:77 (average 1:59) in the US pediatric population across eleven states in 2014 *(*CDC Morbidity and Mortality Weekly Report Surveillance Summaries, April 27, 2018 / 67(6):1-23)*.* Based on a number of epidemiological studies, the median of prevalence estimate of ASD was 62/10,000 in 2012 *(*Elsabbagh et al., Autism Res. 2012, 5:160-179*).* Thus, ASD is recognized today as a global, common, lifelong neurodevelopmental disorder that affects approximately 1% of both children and adults *(*Brugha et al., Arch. Gen. Psychiatry. 2011, 68:459-465; Murphy et al., Neuropsychiatr. Dis. Treat. 2016, 12:1669-1686*).*

The disorder is characterized by two core symptoms of socio-communicational dysfunctions as well as restricted (repetitive, stereotyped) behaviors and thinking *(*Diagnostic and Statistical Manual of Mental Disorders, Fifth Edition, pp. 50-59*).* Social impairments comprise abnormal social approach, failure of normal back-and-forth communication, failure to initiate and reciprocate interaction. Communicational deficits may include poorly integrated verbal and nonverbal communication, abnormal eye contact and body language, deficits in understanding gestures, lack of facial expressions. In general, deficits in developing, maintaining and understanding relationships, adjusting to social situations, sharing imaginative play and absence of interest in peers may be present. With respect to the other core symptom domain, stereotyped or repetitive motor movements, insistence on sameness and routines, highly fixated interests that are abnormal in intensity or focus and abnormal sensory reactivity can be identified.

In addition to the core symptoms, ASD is also often accompanied by associated or comorbid symptoms including intellectual disability, attention deficit, hyperactivity, mood disorders, seizures, sleep problems, etc. A further frequently associated symptom domain is irritability that comprises tantrums, aggression towards others, self-injurious behavior and mood swings.

The unmet medical need in ASD is enormous, since there is no pharmacological treatment currently available for the treatment of core symptoms in ASD. While there is no approved drug for the treatment of core symptoms, only two antipsychotics of the many available drugs of the same class - risperidone and aripiprazole - have been approved in the US Food & Drug Administration for the treatment of ASD-associated irritability in children, ages 5-16 years. Aripiprazole has also been approved for this purpose in Japan. Although large efforts have been put into clinical research, no effective pharmacological treatment has been identified until now to alleviate the core symptom domains of ASD.

Risperidone and aripiprazole are atypical antipsychotics with different receptor profiles in vitro. Whereas risperidone has been found to bind with high affinity to serotonin 5-HT_{2A} receptors and function as an antagonist of the receptor, aripiprazole displays high affinity for dopamine D₂, D₃, 5-HT_{1A} and 5-HT_{2B} receptors, and it behaves as a partial agonist at dopamine D₂, D₃ and serotonin 5-HT_{1A} receptors. *(*Shahid et al., J. Psychopharmacol. 2009, 23:65-73*;* Tadori et al. Eur. J. Pharmacol. 2011, 668:355-365*;* Shapiro et al., Neuropsychopharmacol. 2003, 28:1400-1411*).* It is important to note that both aripiprazole and risperidone occupy dopamine D₂ receptors in the human brain *(*Muly et al., J. Pharmacol. Exp. Ther. 2012, 341:81-89*;* Gründer et al., Am. J. Psychiatry 2008, 165:988-995*).* However, neither of the two compounds were found to demonstrate occupancy of dopamine D₃ receptors in humans *(*Graff-Guerrero et al., Arch. Gen Psychiatry 2009, 66:606-615*).*

Although risperidone and aripiprazole are used to treat ASD-associated irritability, these antipsychotic compounds are neither approved nor used to treat the core symptom domains of ASD. Results of clinical investigations with regards to the usefulness of risperidone and aripiprazole in the treatment of the core symptoms are contradictory, showing lack of efficacy for aripiprazole and mixed results for risperidone. Aripiprazole was investigated in open label trials and it did not improve the lethargy/social withdrawal subscale of the Aberrant Behavioral Checklist *(*Ichikawa et al., Child Psychiary Hum. Dev. 2017, 48:796-806*;* LeClerc et al., Pharma. Ther. 2015, 40:389-397*;* Posey et al., Child Adolesc. Psychiatry Clin. N. Am. 2008, 17:787-798*).* Studies with risperidone suggest that it may result in modest improvements of the core symptoms in children with pervasive developmental disorders exhibiting high levels of baseline irritability however, it is unclear whether risperidone improved these symptoms in the absence of irritability *(*LeClerc et al., Pharma. Ther. 2015, 40:389-397*;* Posey et al., Child Adolesc. Psychiatry Clin. N. Am. 2008, 17:787-798*).* Thus, for aripiprazole there is no support from human studies for its usefulness to treat socio-communicational dysfunction in ASD. In case of risperidone, there is also a lack of solid evidence for social behavioral efficacy in ASD subjects.

Pharmacodynamic effects of risperidone and aripiprazole have also been investigated in animal models of autism, and yielded mixed results. In a number of preclinical ASD mouse models, including the prenatal valproate model *(*Auclair et al., World Congress of The International College of Neuropsychopharmacology 2014, LP-03-011*),* the *Cntnap2* knockout mice *(*Penagarikano et al., Cell 2011, 147:235-246*),* the NMDA receptor NR1 subunit hypomorph mice *(*Teng et al., Neuropharmacology 2016, 105:61-71*)* and the BTBR mouse strain *(*Chadman, Pharm. Biochem. Behav. 2011, 97:586-594*)* neither aripiprazole nor risperidone could improve the social behavior deficits. In another study, utilizing prenatal valproate treatment in mice, both risperidone and aripiprazole improved core behavioral deficits after chronic administration, while acute treatment was without effect *(*Hara et al., Psychopharmacology 2017, 234:3217-3228*).* Hara *et al.* discusses possible mechanisms behind the improvements of ASD-like symptoms due to chronic risperidone and aripiprazole treatment and emphasize the role of dopamine D₁ and D₂ receptor activation following dopamine release in the prefrontal cortex. The authors do not mention any role for dopamine D₃ receptors in the molecular mechanism whereby risperidone or aripiprazole may alleviate the behavioral effects of prenatal valproate treatment.

Patent application WO 2005/012266A1 discloses carbamoyl cyclohexane derivatives that are dopamine D_{3/}D₂ receptor partial agonists. Notably, WO 2005/012266A1 discloses trans-N-[4-[2-[4-(2,3-dichlorophenyl)piperazin-1-yl]ethyl]cyclohexyl]-N',N'-dimethylurea (cariprazine) corresponding to Formula 1 and its hydrochloride salt. As it was later established, cariprazine (US: Vraylar^{®}, Europe: Reagila^{®}) is a dopamine D₃-preferring D_{3/}D₂ receptor and serotonin 5-HT_{1A} receptor partial agonist (Kiss et al., J. Pharmacol. Exp. Therap. 2010, 333:328-340*)* that has been approved to treat schizophrenia (US and Europe) and manic or mixed episodes associated with bipolar I disorder (US). Active metabolites of cariprazine include, not exclusively, desmethyl cariprazine and didesmethyl cariprazine (WO 2008/142461A1). Additional patent applications describe the hydrochloride salt of cariprazine (WO 2008/139235A2) and the deuterized derivative of cariprazine (WO 2011/060363A2). WO 2005/012266A1 also discloses close analogs of cariprazine that are listed on page 21-22 of the said patent application. These close analogs also include trans-N-(4-{2-[4-(2,3-dichloro-phenyl)-piperazine-1-yl]-ethyl}-cyclohexyl)-morpholine-4 carboxamide (Compound 2).

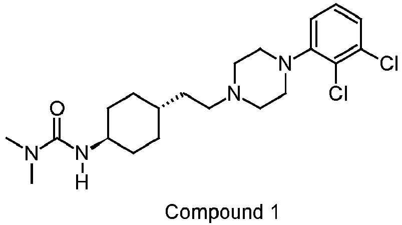

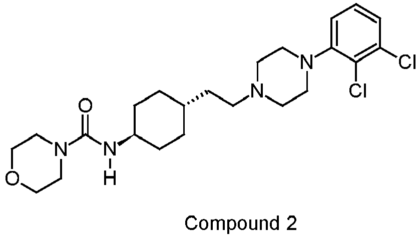

Among the diseases to be treated and/or prevented by the carbamoyl cyclohexane derivatives, WO 2005/012266A1, WO2008/139235A2, WO2008/141135A1, WO2009/020897A1 and WO2010/009309A1 claim the treatment of autism as such. As mentioned above, ASD is a very complex disorder with regard to its manifestation in human patients; its core symptoms include socio-communicational impairments and restricted/repetitive behaviors with variable severity as well as a great variety of comorbid symptoms. The selected ASD-associated symptoms defined hereafter were neither specified nor supported by any scientific evidence in the aforementioned patent applications. These symptoms specifically include socio-communicational impairments, restricted and repetitive behaviors, attention deficit and hyperactivity, as well as irritability.

Compared to risperidone and aripiprazole, cariprazine has a unique receptor profile *(*Shahid et al., J. Psychopharmacol. 2009, 23:65-73*;* Kiss et al., J. Pharmacol. Exp. Therap. 2010, 333:328-340*).* Cariprazine shows subnanomolar affinity for the dopamine D₃, D_{2L} and D_{2S} receptors (Kᵢ values of 0.09, 0.5 and 0.7 nM, respectively) with an approximately 8-fold preference for the dopamine D₃ versus D₂ receptor. Functionally, the compound is a partial agonist at the dopamine D₃ and D₂ receptors. In addition, cariprazine shows nanomolar affinity for the serotonin 5-HT_{2B} and 5-HT_{1A} receptors (Kᵢ 0.6 and 2.6 nM, respectively) where it acts as full antagonist and weak partial agonist, respectively. The close analog of cariprazine (Compound 2) shows a very similar molecular pharmacological profile to cariprazine. It has also subnanomolar affinity for the dopamine D₃, D_{2L} and D_{2S} receptors (Kᵢ 0.17, 0.4 and 0.52 nM, respectively). Functionally, Compound 2 is a partial agonist at the dopamine D₃ and D₂ receptors. In addition, Compound 2 shows nanomolar affinity for the serotonin 5-HT_{2B} and 5-HT_{1A} receptors (Kᵢ 1.11 and 5.73 nM, respectively), where it acts as full antagonist and weak partial agonist, respectively. When taking into account this combination of receptorial affinities and functionalities, cariprazine and its close analog (Compound 2) are special and clearly different from other available antipsychotics.

Although results of genetic association studies available at that time *(*Martineau et al., Dev. Med. Child Neurol. 1994, 36:688-697*;* Persico and Napolioni, Behav. Brain Res. 2013, 251:95-112*)* did not imply the dopamine D₃ receptors in the pathomechanism of ASD, researchers of Pierre Fabre Medicament investigated as first ones a dopamine D₃ receptor antagonist and found it effective in an animal model of ASD. Pierre Fabre Medicament filed a patent application (WO 2015/086836A1) related to a chromone derivative for its use as medicament for the treatment of ASD. The chromone derivative in WO 2015/086836A1 is a dopamine D₃ receptor full antagonist lacking any intrinsic activity revealed by a MAP kinase activity test on human recombinant dopamine D₃ receptors. The chromone derivative of that invention was shown to improve impaired social behavior of rats that had been exposed to sodium valproate during their intrauterine life. Despite its beneficial effects in the above animal model of ASD, there is no available clinical evidence up to now that the chromone compound disclosed in the patent application WO 2015/086836A1 or related compounds are useful for the treatment of this disorder in human patients.

Whereas cariprazine is a partial agonist at dopamine D₂ and D₃ receptors *(*Kiss et al., J. Pharmacol. Exp. Therap. 2010, 333:328-340*;* Tadori et al. Eur. J. Pharmacol. 2011, 668:355-365*),* and according to WO 2015/086836A1 the chromone derivatives by Pierre Fabre Medicament patent are defined as dopamine D₃ antagonists, therefore a skilled person in the art would not consider the two compounds to have similar effect.

First, there is clear difference between the chemical structures. The structural differences between the two base compounds have consequences on differences in physicochemical properties, molecular pharmacological, pharmacodynamic and pharmacokinetic properties.

Second, while the Pierre Fabre compound produced no stimulatory activity in a cellular system (ERK1/2 phosphorylation assay) *(*Heusler et al., Eur. Neuropsychopharm. 2016, 26(S2):S490-S491*),* cariprazine stimulated ERK1/2 phosphorylation in Chinese hamster ovary cells expressing the D₃ receptor *(unpublished data, data in company file).* This implies that the two compounds behave completely differently in *in vitro* functional assays.

Third, the two compounds have different receptor profiles *(*Heusler et al., Eur. Neuropsychopharm. 2016, 26(S2):S490-S491*;* Kiss et al., J. Pharmacol. Exp. Therap. 2010, 333:328-340*).* The Pierre Fabre chromone derivative has subnanomolar affinity for the dopamine D₃ receptor (Kᵢ 0.16 nM), nanomolar affinities for the dopamine D_{2L} and D_{2S} receptors (Ki 12.6 and 6.3 nM, respectively). Thus, there is a greater preference for the dopamine D₃ receptors versus D₂ receptors in case of the Pierre Fabre compound than in case of cariprazine. The chromone derivative has considerable serotonin 5-HT_{1A} receptor affinity (Ki 0.13 nM), while cariprazine shows lower affinity for this receptor (Ki 2.6 nM). Cariprazine, its close analog (Compound 2) and the Pierre Fabre compound are all partial agonists of the serotonin 5-HT_{1A} receptor however, while the Pierre Fabre chromone derivative has substantial intrinsic activity (57-75%) *(*Heusler et al., Eur. Neuropsychopharm. 2016, 26(S2):5490-5491*),* both cariprazine and Compound 2 are only weak partial agonists with intrinsic activities of 38 and 30 %, respectively (Kiss et al., J. Pharmacol. Exp. Therap. 2010, 333:328-340*, and unpublished adat, data in company file).*

As mentioned above, cariprazine has a special receptor profile comprising dopamine D_{3/}D₂ receptor partial agonism, weak serotonin 5-HT_{1A} receptor partial agonism and serotonin 5-HT_{2B} receptor full antagonism. Lim *et al.* found that treatment of *Fmr1* knock-out mice with the selective serotonin 5-HT_{2B} receptor agonist BW723C86 resulted in the improvement of cognitive abilities *(*Lim et al., Gene. Dev. 2014, 28:273-289*).* Further studies reported that there is also reduced function of serotonin 5-HT₂ type (not-specified) receptors in the periphery and brain of ASD patients *(*McBride et al., Arch. Gen. Psychiatry 1989, 46, 213-221*).* Taking the above results into account it is not expected that a serotonin 5-HT_{2B} receptor antagonist such as cariprazine would be effective in an animal model of ASD.

Moreover, Oblak et al. (Autism Res. 2013, 6:571-583*)* found significantly reduced serotonin 5-HT_{1A} receptor binding in *post mortem* cortical samples of subjects with autism in two regions of a limbic-cortical network that contribute to social-emotional behaviors. Given these reduced serotonin 5-HT_{1A} receptor levels, it is unlikely that a weak partial agonist such as cariprazine at these receptors would be effective in an ASD model.

Along with the need to treat autism in general, there is also a need to treat one or more symptoms of autism. Furthermore, there is a need to treat conditions such as Asperger's syndrome, atypical autism (otherwise known as pervasive developmental disorder not otherwise specified; PDD-NOS), Rett syndrome, childhood disintegrative disorder, attention deficit hyperactivity disorder (ADHD) and sensory integration dysfunction.

### SUMMARY OF THE INVENTION

The present application relates to trans-N-[4-[2-[4-(2,3-dichlorophenyl)piperazin-1-yl]ethyl]cyclohexyl]-N',N'-dimethylurea (cariprazine), and/or its close analog trans-N-[4-[2-[4-(2,3-dichlorophenyl)piperazin-1-yl] ethyl] cyclohexyl]-morpho line-4 carboxamide and/or its deuterated derivatives and/or pharmaceutically acceptable salts thereof, as well as pharmaceutical compositions comprising them, for use in the treatment of a core symptom of autism spectrum disorder, wherein core symptom of autism spectrum disorder are socio-communicational deficits or restricted and repetitive behaviors; or for use in the treatment of irritability associated with autism spectrum disorder; or for use in the treatment of attention deficit and hyperactivity associated with autism spectrum disorder.

### DETAILED DESCRIPTION OF THE INVENTION

The present invention is defined by the claims. Any subject-matter falling outside the scope of the claims is provided for information only. Methods of treatment of the human body by therapy, referred to in this description, are not part of the present invention as such, but are described here in relation to compounds and pharmaceutical compositions for use in said methods for treatment of the human body by therapy, according to the present invention.

In one aspect, the present application relates to trans-N-[4-[2-[4-(2,3-dichlorophenyl)piperazin-1-yl]ethyl]cyclohexyl]-N',N'-dimethylurea hydrochloride (cariprazine) and trans-N-(4-{2-[4-(2,3-dichloro-phenyl)-piperazine-1-yl]-ethyl}-cyclohexyl)-morpholine-4 carboxamide hydrochloride (Compound 2) disclosed in WO 2005/012266A1. Cariprazine showed great benefit in an animal model for the symptoms of ASD. These result indicate that cariprazine, its close analogs and its derivatives may be of therapeutic use against the symptoms of ASD in human patients.

In one aspect, the application relates to cariprazine, its pharmaceutically acceptable salts, the close analog as defined above, deuterated derivatives, pharmaceutical compositions, and combinations for use in the treatment of the symptoms of autism spectrum disorder as defined below.

In a further aspect, the autism spectrum disorder is selected from Asperger's syndrome, atypical autism (otherwise known as pervasive developmental disorder not otherwise specified; PDD-NOS), and childhood disintegrative disorder.

In a preferred embodiment, cariprazine or a pharmaceutically acceptable salt thereof is for use in the treatment of persistent deficits in social communication and social interactions associated with ASD; for the treatment of restrictive or repetitive behaviors associated with ASD; for the treatment for stereotyped or repetitive movements associated with ASD; and/or as a treatment for deficits in social-emotional reciprocity associated with ASD.

In a preferred embodiment, cariprazine or a pharmaceutically acceptable salt thereof is for use in the treatment of one or more symptoms of ASD selected from social communication deficits, restricted interests and repetitive behaviors. In some embodiments, cariprazine or a pharmaceutically acceptable salt thereof is for use in the treatment of one or more symptoms of ASD selected from restricted, repetitive, and stereotyped patterns of behaviors, interests and activities.

Cariprazine and Compound 2 were investigated in the prenatal valproate model of ASD. As described in the Examples, cariprazine hydrochloride and Compound 2 hydrochloride were able to reverse behavioral deficits in rats that had been exposed to valproate during their intrauterine life. The results show that the above two compounds are suitable to treat symptoms of ASD.

In another preferred embodiment, the present application is directed to trans-N-[4-[2-[4-(2,3-dichlorophenyl)piperazin-1-yl]ethyl]cyclohexyl]-N',N'-dimethylurea and/or its close analog as defined above and/or its deuterated derivatives and/or pharmaceutically acceptable salts thereof for use in the treatment of socio-communicational deficits as core symptom of autism spectrum disorder.

In another preferred embodiment, the present application is directed to trans-N-[4-[2-[4-(2,3-dichlorophenyl)piperazin-1-yl]ethyl]cyclohexyl]-N',N'-dimethylurea and/or its close analog as defined above and/or its deuterated derivatives and/or pharmaceutically acceptable salts thereof for use in the treatment of restricted and repetitive behaviors as core symptom of autism spectrum disorder.

In another preferred embodiment, the present application is directed to trans-N-[4-[2-[4-(2,3-dichlorophenyl)piperazin-1-yl]ethyl]cyclohexyl]-N',N'-dimethylurea and/or its close analog as defined above and/or its deuterated derivatives and/or pharmaceutically acceptable salts thereof for use in the treatment of irritability associated with autism spectrum disorder.

In another preferred embodiment, the present application is directed to trans-N-[4-[2-[4-(2,3-dichlorophenyl)piperazin-1-yl]ethyl]cyclohexyl]-N',N'-dimethylurea and/or its close analog as defined above and/or its deuterated derivatives and/or pharmaceutically acceptable salts thereof for use in the treatment of attention deficit and hyperactivity associated with autism spectrum disorder.

In another preferred embodiment the application relates to a pharmaceutical composition defined above, wherein the core symptoms of autism spectrum disorder are socio-communicational deficits. In another preferred embodiment the application relates to a pharmaceutical composition defined above, wherein the core symptoms the core symptoms of autism spectrum disorder are restricted and repetitive behaviors.

In another preferred embodiment the application relates to a pharmaceutical composition defined above, wherein the condition to be treated is irritability associated with autism spectrum disorder.

In another preferred embodiment the application relates to a pharmaceutical composition defined above, wherein the condition to be treated is attention deficit and hyperactivity associated with autism spectrum disorder.

The compositions according to the present application can be administered by the oral, transdermic, parenteral, intranasal and rectal routes. The compositions can especially be administered by the oral route in an appropriate formulation. The dosages of the compound (trans-N-[4-[2-[4-(2,3-dichlorophenyl)piperazin-1-yl]ethyl]cyclohexyl]-N',N'-dimethylurea) or its close analog as defined above, pharmaceutically acceptable salts, or deuterated derivatives in the compositions of the application can be adjusted to obtain an amount of active substance that results in the desired therapeutic response. Therefore, the dosage level depends on the desired therapeutic response, the route of administration, the expected duration of treatment and other factors such as age, gender or body weight of the patient. The dosages can be from 0.01 to 12 mg daily and can be titrated to effect.

Cariprazine may also be used in combination with at least one other active ingredient that have been already in use for the treatment of comorbid symptoms of ASD (e.g., psychostimulants, antipsychotics, antidepressants, anxiolytics, antihypertensives, antiepileptics, narcotics, spasmolytics or other agents).

Psychostimulants include, but not limited to, centrally acting sympathomimetics (amphetamine, methylphenidate, modafinil, atomoxetine), nootropics or other psychostimulants (vinpocetine, donepezil, memantine).

Antipsychotics include, but not limited to, typical and atypical antipsychotics, such as haloperidol, pimozide, clozapine, olanzapine, quetiapine, sertindole, ziprasidone, lurasidone, risperidone, aripiprazole, brexpiprazole, iloperidone, paliperidone, lithium.

Antidepressants include, but not limited to, non-selective monoamine reuptake inhibitors (desipramine, imipramine, clomipramine, amitriptyline, nortriptyline), serotonin modulator and stimulators (vilazodone, vortioxetine), selective serotonin reuptake inhibitors (fluoxetine, paroxetine, sertraline, fluvoxamine, citalopram, escitalopram), non-hydrazide monoamine oxidase inhibitor (moclobemide,) or other agents (mianserin, trazodone, nefazodone, mirtazapine, tianeptine, venlafaxine, milnacipran, reboxetine, duloxetine, agomelatine, bupropion, gepirone).

Anxiolytics include, but not limited to, benzodiazepines (diazepam, chlorodiazepoxide, oxazepam, lorazepam, alprazolam), azaspirode-diones (buspirone).

Antihypertensives include, but not limited to, imidazoline receptor agonists (clonidine, guanfacine) and a combination of these substances with a diuretic.

Antiepileptics include, but not limited to, barbiturates and their derivatives (phenobarbital), hydantoin derivatives (phenytoin), succinimide derivatives (ethosuximide), benzodiazepine derivative clonazepam, carboxamide derivatives (carbamazepine, oxcarbazepine), fatty acid derivatives (valproic acid, valpromide, vigabatrin, tiagabine) and other antiepileptics (lamotrigine, topiramate, gabapentin, levetiracetam, zonisamide, pregabalin).

Narcotics include, but not limited to, barbiturates (pentobarbital), benzodiazepines (midazolam), cyclopyrrolone benzodiazepine derivatives (zopiclone, zolpidem), melatonin receptor agonists (melatonin, ramelteon).

Spasmolytics or antispasmodics include, but not limited tocentrally acting agents (baclofen, arbaclofen, tolperisone) and papaverine.

Other agents include, but not limited to, medicinal products (probiotics, digestive aids/digestives, herbal extracts), vitamins (both water soluble and fat soluble, such as, but not limited to, vitamin A, D3, E, K, B1, B5, B6, B12, C or their derivatives) and nutritional supplements (coenzymes eg. Q10, flavonoids eg., resveratrol, lecithin, unsaturated fatty acids, including fatty acids ω3 and ω6).

Accordingly, the present invention also relates to a pharmaceutical composition for use in treating autism spectrum disorder comprising
1) trans-N-[4-[2-[4-(2,3-dichlorophenyl)piperazin-1-yl]ethyl]cyclohexyl]-N',N'-dimethylurea and/or its close analog trans-N-[4-[2-[4-(2,3-dichlorophenyl)piperazin-1-yl] ethyl] cyclohexyl]-morpho line-4 carboxamide and/or its deuterated derivatives and/or pharmaceutically acceptable salts thereof
   and
2) at least one adjunctive therapeutical agent, which is selected from the group consisted of psychostimulants/nootropics, antipsychotics, antidepressants, anxiolytics, antihypertensives, antiepileptics, narcotics and spasmolytics and
3) one or more pharmaceutically acceptable carriers, diluents and excipients.

At the same time, the present application also relates to a pharmaceutical combination comprising trans-N-[4-[2-[4-(2,3-dichlorophenyl)piperazin-1-yl]ethyl]cyclohexyl]-N',N'-dimethylurea and/or its close analog trans-N-[4-[2-[4-(2,3-dichlorophenyl)piperazin-1-yl] ethyl] cyclohexyl]-morpho line-4 carboxamide and/or its deuterated derivatives and/or pharmaceutically acceptable salts thereof and at least one adjunctive therapeutical agent for use in treating autism spectrum disorder. In a preferred embodiment the present application relates to a pharmaceutical combination defined above, wherein at least one adjunctive therapeutical agent is selected from the group consisted of psychostimulants/nootropics, antipsychotics, antidepressants, anxiolytics, antihypertensives, antiepileptics, narcotics and spasmolytics.

In another preferred embodiment the present application relates to a pharmaceutical combination defined above, wherein psychostimulants/nootropics are selected from the list comprising amphetamine, methylphenidate, modafinil, atomoxetine, vinpocetine, donepezil and memantine.

In another preferred embodiment the present application relates to a pharmaceutical combination defined above, wherein antipsychotics are selected from the list comprising haloperidol, pimozide, clozapine, olanzapine, quetiapine, sertindole, ziprasidone, lurasidone, risperidone, aripiprazole, brexpiprazole, iloperidone, paliperidone and lithium.

In another preferred embodiment the present application relates to a pharmaceutical combination defined above, wherein antidepressants are selected from the list comprising desipramine, imipramine, clomipramine, amitriptyline, nortriptyline, vilazodone, vortioxetine, fluoxetine, paroxetine, sertraline, fluvoxamine, citalopram, escitalopram moclobemide, mianserin, trazodone, nefazodone, mirtazapine, tianeptine, venlafaxine, milnacipran, reboxetine, duloxetine, agomelatine, bupropion and gepirone.

In another preferred embodiment the present application relates to a pharmaceutical combination defined above, wherein anxiolytics are selected from the list comprising diazepam, chlorodiazepoxide, oxazepam, lorazepam, alprazolam and buspirone.

In another preferred embodiment the present application relates to a pharmaceutical combination defined above, wherein antihypertensive is selected from clonidine and guanfacine.

In another preferred embodiment the present application relates to a pharmaceutical combination defined above, wherein antiepileptics are selected from the list comprising phenobarbital, phenytoin, ethosuximide, clonazepam, carbamazepine, oxcarbazepine, valproic acid, valpromide, vigabatrin, tiagabine, lamotrigine, topiramate, gabapentin, levetiracetam, zonisamide and pregabalin.

In another preferred embodiment the present application relates to a pharmaceutical combination defined above, wherein narcotics are selected from the list comprising pentobarbital, midazolam, zopiclone, zolpidem, melatonin and ramelteon.

In another preferred embodiment the present application relates to a pharmaceutical combination defined above, spasmolytics are selected from the list comprising baclofen, arbaclofen, tolperisone and papaverine.

In one embodiment, cariprazine or a pharmaceutically acceptable salt thereof, is used for the treatment of a pediatric patient of age between about 5 to 12; or an adolescent patient of age between about 13 to 17; or an adult patient of age about 18 or above.

### Preparation of pharmaceutical compositions

The following formulation examples illustrate representative pharmaceutical compositions of this application. The present application includes the following pharmaceutical compositions.
A. Solid oral dosage forms
   a. Tablets
   b. Capsules
   c. Granules
B. Liquid oral dosage forms
   a. Syrups
C. Other dosage forms
   a. Suppositories
   b. Transdermal patches
   c. Nasal sprays, aerosols

### DEFINITIONS

The term "affinity" means the attraction of a drug for a biological target; it is a chemical term used to quantify the strength of drug-target interaction.

The term "full agonist" means a compound that produces the full maximal response of the biological system.

The term "partial agonist" means a compound that associates with a receptor and have only partial efficacy at the receptor relative to a full agonist.

The term "antagonist" means a compound that associates with a receptor and produces no response or prevents the response generated by an agonist of the same receptor.

The term "close analog" means compounds that are related to a compound based on their similarity of chemical structure as well as their in vitro pharmacological profile.

The term "salt" means nontoxic base addition salts of the compounds of the invention which are generally prepared by reacting the acid with a suitable organic or inorganic base.

The term "active metabolite" means such metabolites produced by different routes of biotransformation whose biological activity is similar to that of the parent compound.

The term "derivative" means such compounds that have been produced by chemical modification of cariprazine and its close analogs resulting not exclusively in prodrugs, deuterated compounds, bioisosters, etc.

The term "active ingredient" means cariprazine, its close analogs, salts, active metabolites and derivatives.

The term "pharmaceutically acceptable" describes an ingredient that is useful in preparing a pharmaceutical composition and is generally safe, non-toxic and neither biologically nor otherwise undesirable, and includes those acceptable for human pharmaceutical use.

The term "pharmaceutical composition" refers to a mixture of a compound of the invention with other chemical components, such as pharmaceutically acceptable excipients e.g. diluents or carriers. The pharmaceutical composition facilitates administration of the compound to the subject.

The term "excipient" defines a chemical compound that facilitates the incorporation of a compound into cells or tissues.

"Core symptoms" include socio-communicational dysfunctions and as well as restricted (repetitive, stereotyped) behaviors and thinking as described in the Diagnostic and Statistical Manual of Mental Disorders, Fifth Edition (2013).

The term "comorbid symptoms" means symptoms that may be associated with ASD, but are not core symptoms according to the Diagnostic and Statistical Manual of Mental Disorders, Fifth Edition (2013). As used herein, the term "treatment" means using an effective therapy to reduce, alleviate or eliminate the symptoms associated with ASD.

The term "patient" refers to a human who received an ASD diagnosis.

### The prenatal valproate model

Prenatal valproate (valproic acid, VPA) exposure is known to increase the risk of ASD in humans *(*Christensen et al, JAMA 2013, 309*).* VPA exposure during intrauterine life in rodents is also known to lead to autistic-like phenotype in the offspring *(*Roullet et al, Neurotox. Teratol. 2013, 36, 45-56*).* VPA in rodents is administered typically around the 12th day of embryonic development when the closure of the neural tube and establishment of cranial nerve nuclei and the cerebellum occurs. The histone deacetylase inhibitory effect of VPA interferes with the above mentioned developmental steps and thereby brings about the autistic-like phenotype in the offspring. The autistic-like phenotype comprises - not exclusively - impaired communication in rat pups, impaired social play behavior in adolescent rats, hyperactivity, excessive stereotypical behavior and defective sociability in the 3-chamber assay in adult rats. Since the physiological origin and the symptoms of the prenatal VPA model show a good match with the human condition, this is a widely accepted rodent model of ASD with high translational value.

As described above, the prenatal VPA model has excellent construct and face validity, therefore it is a widely accepted disease model of ASD. In this method, time-mated female Wistar rats are administered a single dose of VPA (300-600 mg/kg, i.p.) on gestational day 12.5. Offspring are housed according to standard laboratory conditions until time of behavioral testing. Animals are housed in groups of 4 in conventional cages and maintained at 22-24°C on a standard 12 hour light/dark cycle, with food and water available ad libitum. After investigational drug treatment, offspring are examined behaviorally in tests relevant for the assessment of autistic behavior. These tests include maternal deprivation-induced ultrasonic vocalization in rat pups, social play, social preference and the open field.

Maternal deprivation-induced ultrasonic vocalization in rat pups is a readout of socio-communicational function that is impaired in offspring after prenatal exposure to VPA *(*Gandal et al., Biol. Psychiatry 2010, 68, 1100-1106). To induce ultrasonic calls, prenatally VPA treated rat pups are placed individually into a cage where calls are being recorded with bat microphones. Calls are digitized with an audio filter and ultrasonic vocalization is recorded and quantified with SonoTrack software (Metris bv. The Netherlands). Baseline vocalization is measured on postnatal days 11-12 days for 10 min. Animals are divided into homogenous groups based on baseline vocalizations. On postnatal day 13, animals are treated p.o. with the appropriate doses of drugs or vehicle 60 min before measurement and then returned to their nests until recording. Ultrasonic call counts are recorded for 10 min. Statistical analysis is performed on ultrasonic call counts with the non-parametric Kruskal-Wallis test and the post hoc Dunnett test.

Social play is a type of social interaction that is highly typical of adolescent mammals including rodents as well as humans *(*Vanderschuren and Trezza, Curr. Topics Behav. Neurosci. 2014, 16:189-212*).* Social play behavior is indicative of social functioning in adult life and is defective after prenatal VPA treatment in the offspring *(*Schneider and Przewlocki Neuropsychopharmacology 2005, 30:80-89*).* At postnatal day 30, following 8 days treatment with the test compound, prenatally VPA-treated rats are evaluated for juvenile play behavior. The test is carried out in a novel test arena with pairs of animals from the same treatment group over a 15-min trial. Animals are scored for play activity measured by duration of social play behavior. Statistical analysis employs one-way ANOVA and the post hoc Dunnett test.

The social preference in a 3-chamber apparatus is another indicator of intact social behavior in rats. The preference of a conspecific over an inanimate object as well as the ability to distinguish between familiar and novel conspecifics are necessary for normal social functioning and are defective in the prenatal VPA model in rats (Bambini-Junior et al., Brain Res. 2011, 1408:8-16). Social preference and social recognition memory are investigated in a 3-chamber apparatus. On postnatal day 59, prenatally VPA-treated rats are assessed for their social preference following 8 days of per os treatment with the test compound. On postnatal day 60, the same rats are assessed for their social recognition memory after 9 days of per os treatment with the test compound. Statistical analysis is performed by using two-way ANOVA, Student's t test and Dunnett test.

Beside socio-communicational impairments, repetitive behaviors are the other core symptom domain of ASD. Hyperactivity and excessive repetitive behaviors have been found in animals that were exposed to VPA during their intrauterine life *(*Schneider and Przewlocki Neuropsychopharmacology 2005, 30:80-89). At postnatal day 31-32, animals are scored for locomotor activity and exploratory behavior in a 10-min trial in an open field test arena after 9-day oral treatment with the test compound. Activity is analysed for readouts of repetitive/stereotypic behaviors such as total distance moved and frequency of circling behavior. Statistical analysis employs one-way ANOVA and the post hoc Dunnett test.

### DESCRIPTION OF FIGURES

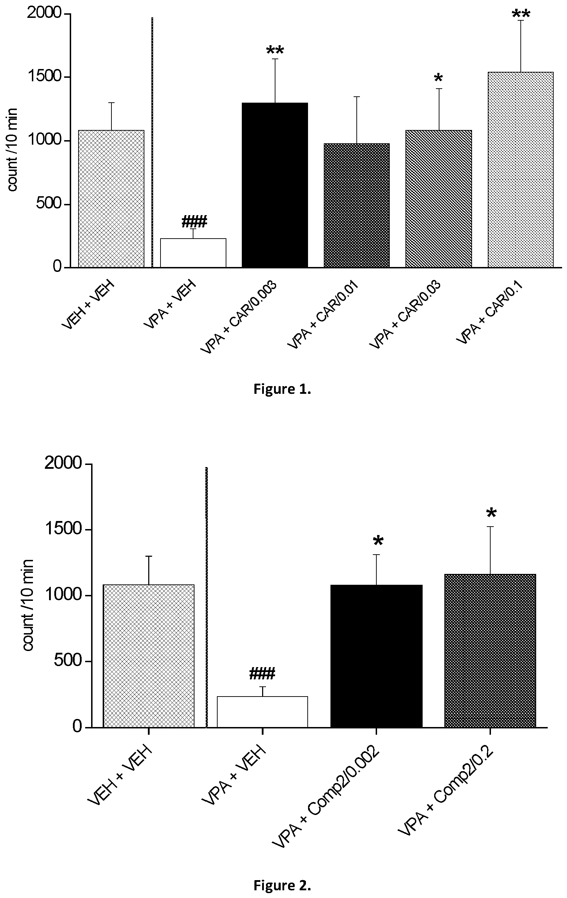

**Figure 1****.** Acute per os administration of trans-N-[4-[2-[4-(2,3-dichlorophenyl)piperazin-1-yl]ethyl]cyclohexyl]-N',N'-dimethylurea hydrochloride (cariprazine) reversed the socio-communicational deficit induced by prenatal valproate treatment (### p<0.001; Dunnett multiple comparisons test versus "VPA + VEH"). The effect of cariprazine was statistically significant at the doses of 0.003, 0.03 and 0.1 mg/kg (*, ** p<0.05, <0.01; Dunnett test versus "VPA + VEH"). VEH + VEH means prenatal vehicle treatment combined with vehicle treatment before behavioral testing. VPA + VEH animals received prenatal VPA combined with vehicle treatment before behavioral testing. VPA + CAR/0.003, CAR/0.01, CAR/0.03 and CAR/0.1 groups received prenatal VPA followed by 0.003, 0.01, 0.03 and 0.1 mg/kg cariprazine, respectively.
**Figure 2****.** Acute per os administration of trans-N-(4-{2-[4-(2,3-dichloro-phenyl)-piperazine-1-yl]-ethyl}-cyclohexyl)-morpholine-4 carboxamide hydrochloride (Compound 2) reversed the socio-communicational deficit induced by prenatal valproate treatment (### p<0.001; Dunnett multiple comparisons test versus "VPA + VEH"). The effect of Compound 2 was statistically significant at both investigated doses (* p<0.05; Dunnett test versus "VPA + VEH"). VEH + VEH means prenatal vehicle treatment combined with vehicle treatment before behavioral testing. VPA + VEH animals received prenatal VPA combined with vehicle treatment before behavioral testing. VPA + Comp2/0.002, and Comp2/0.2 groups received prenatal VPA followed by 0.002 and 0.2 mg/kg Compound2, respectively.

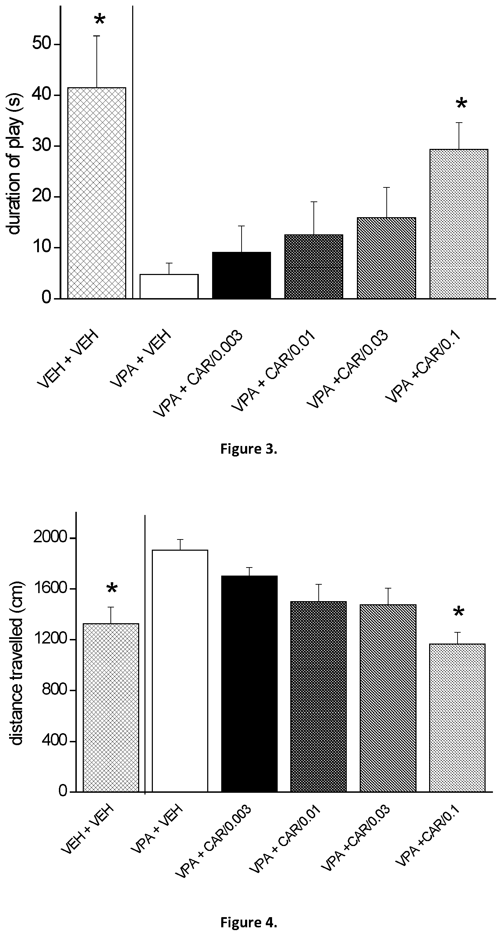

**Figure 3****.** Repeated (daily treatments for 8 days) per os administration of trans-N-[4-[2-[4-(2,3-dichlorophenyl)piperazin-1-yl]ethyl]cyclohexyl]-N',N'-dimethylurea hydrochloride (cariprazine) reversed the social play deficit induced by prenatal valproate treatment. The effect of cariprazine was statistically significant at the dose of 0.1 mg/kg (* p<0.05; Dunnett test versus "VPA + VEH"). VEH + VEH means prenatal vehicle treatment combined with vehicle treatment before behavioral testing. VPA + VEH animals received prenatal VPA combined with vehicle treatment before behavioral testing. VPA + CAR/0.003, CAR/0.01, CAR/0.03 and CAR/0.1 groups received prenatal VPA followed by 0.003, 0.01, 0.03 and 0.1 mg/kg cariprazine, respectively.
**Figure 4****.** Repeated (daily treatments for 9 days) per os administration of trans-N-[4-[2-[4-(2,3-dichlorophenyl)piperazin-1-yl]ethyl]cyclohexyl]-N',N'-dimethylurea hydrochloride (cariprazine) reversed the hyperactivity induced by prenatal valproate treatment. The effect of cariprazine was statistically significant at the dose of 0.1 mg/kg (* p<0.05; Dunnett test versus "VPA + VEH"). VEH + VEH means prenatal vehicle treatment combined with vehicle treatment before behavioral testing. VPA + VEH animals received prenatal VPA combined with vehicle treatment before behavioral testing. VPA + CAR/0.003, CAR/0.01, CAR/0.03 and CAR/0.1 groups received prenatal VPA followed by 0.003, 0.01, 0.03 and 0.1 mg/kg cariprazine, respectively.

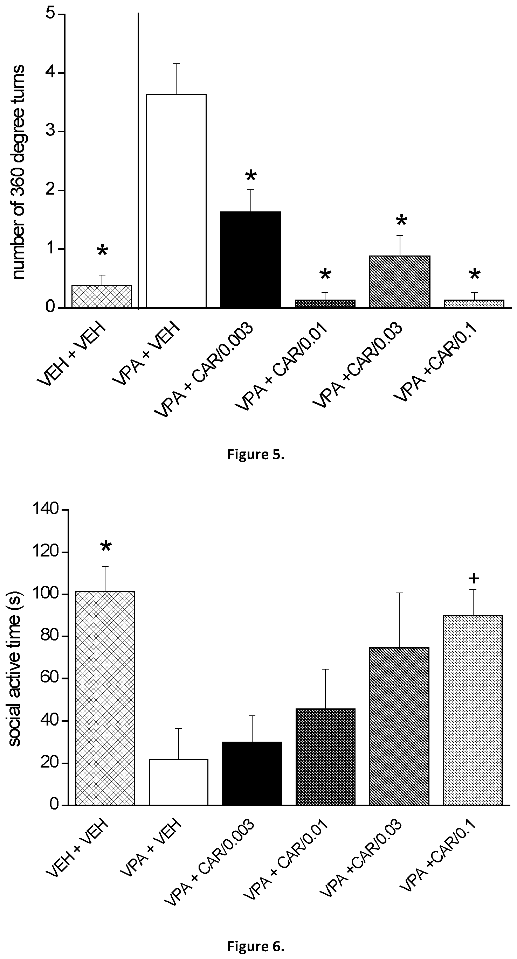

**Figure 5****.** Repeated (daily treatments for 9 days) per os administration of trans-N-[4-[2-[4-(2,3-dichlorophenyl)piperazin-1-yl]ethyl]cyclohexyl]-N',N'-dimethylurea hydrochloride (cariprazine) reversed the excessive circling behavior induced by prenatal valproate treatment. The effect of cariprazine was statistically significant at all doses of cariprazine (* p<0.05; Dunnett test versus "VPA + VEH"). VEH + VEH means prenatal vehicle treatment combined with vehicle treatment before behavioral testing. VPA + VEH animals received prenatal VPA combined with vehicle treatment before behavioral testing. VPA + CAR/0.003, CAR/0.01, CAR/0.03 and CAR/0.1 groups received prenatal VPA followed by 0.003, 0.01, 0.03 and 0.1 mg/kg cariprazine, respectively.
**Figure 6****.** Repeated (daily treatments for 8 days) per os administration of trans-N-[4-[2-[4-(2,3-dichlorophenyl)piperazin-1-yl]ethyl]cyclohexyl]-N',N'-dimethylurea hydrochloride (cariprazine) reversed the social preference deficit induced by prenatal valproate treatment. The effect of cariprazine was statistically significant at the dose of 0.1 mg/kg (* p<0.05; Dunnett test versus "VPA + VEH"). VEH + VEH means prenatal vehicle treatment combined with vehicle treatment before behavioral testing. VPA + VEH animals received prenatal VPA combined with vehicle treatment before behavioral testing. VPA + CAR/0.003, CAR/0.01, CAR/0.03 and CAR/0.1 groups received prenatal VPA followed by 0.003, 0.01, 0.03 and 0.1 mg/kg cariprazine, respectively.

The following examples illustrate the invention without limiting the scope thereof.

### EXAMPLE 1

Trans-N-[4-[2-[4-(2,3-dichlorophenyl)piperazin-1-yl]ethyl]cyclohexyl]-N',N'-dimethylurea hydrochloride (cariprazine) was tested on rat pup communication in prenatally valproate-treated rats. Rat pup ultrasonic vocalization (USV) was evoked by maternal deprivation. The effect of acute administration of cariprazine on rat pup communication is shown in Figure 1. The data represented are mean number of USV vocalizations ± SEM of 13-24 rat pups for each group (both male and female pups were included). Cariprazine given orally increased and fully reversed isolation-induced vocalizations in valproate-treated offspring at the doses of 0.003, 0.03 and 0.1 mg/kg. Thus, cariprazine was able to reduce the socio-communicational deficit induced by prenatal valproate treatment.

### EXAMPLE 2

Trans-N-(4-{2-[4-(2,3-dichloro-phenyl)-piperazine-1-yl]-ethyl}-cyclohexyl)-morpholine-4 carboxamide hydrochloride (Compound 2) was tested on rat pup communication in prenatally valproate-treated rats. Rat pup ultrasonic vocalization (USV) was evoked by maternal deprivation. The effect of acute oral administration of Compound 2 on rat pup communication is shown in Figure 2. The data represented are mean number of USV vocalizations ± SEM of 13-24 rat pups for each group (both male and female pups were included). Compound 2 given orally increased and fully reversed isolation-induced vocalizations in valproate-treated offspring at each dose examined. Thus, Compound 2 was able to reduce the socio-communicational deficit induced by prenatal valproate treatment.

### EXAMPLE 3

Trans-N-[4-[2-[4-(2,3-dichlorophenyl)piperazin-1-yl]ethyl]cyclohexyl]-N',N'-dimethylurea hydrochloride (cariprazine) was tested on rat social play behavior in prenatally valproate-treated rats. The effect of repeated dosing of cariprazine is shown in Figure 3. The data presented are mean time spent playing ± SEM of 4 pairs of rats for each group (only male offspring tested). Cariprazine administered orally increased and partially nevertheless, significantly reversed the social play deficit induced by prenatal valproate treatment. Therefore, cariprazine was able to reduce the socio-communicational deficit induced by prenatal valproate treatment.

### EXAMPLE 4

Trans-N-[4-[2-[4-(2,3-dichlorophenyl)piperazin-1-yl]ethyl]cyclohexyl]-N',N'-dimethylurea hydrochloride (cariprazine) was tested on rat motility in the open field assay in prenatally valproate-treated rats. The effect of repeated dosing of cariprazine is shown in Figure 4. The data presented are mean distance travelled ± SEM of 8 rats for each group (only male offspring tested). Cariprazine administered orally reduced and fully reversed the excessive motility induced by prenatal valproate treatment. Therefore, cariprazine was able to reduce the hyperactivity induced by prenatal valproate treatment.

### EXAMPLE 5

Trans-N-[4-[2-[4-(2,3-dichlorophenyl)piperazin-1-yl]ethyl]cyclohexyl]-N',N'-dimethylurea hydrochloride (cariprazine) was tested on rat stereotypic behavior in the open field assay in prenatally valproate-treated rats. The effect of repeated dosing of cariprazine is shown in Figure 5. The data presented are mean number of 360-degree turns ± SEM of 8 rats for each group (only male offspring tested). Cariprazine administered orally reduced and fully reversed the excessive circling behavior induced by prenatal valproate treatment. Therefore, cariprazine was able to reduce the stereotypic behavior induced by prenatal valproate treatment.

### EXAMPLE 6

Trans-N-[4-[2-[4-(2,3-dichlorophenyl)piperazin-1-yl]ethyl]cyclohexyl]-N',N'-dimethylurea hydrochloride (cariprazine) was tested on rat social preference behavior in the 3-chamber assay in prenatally valproate-treated rats. The effect of repeated dosing of cariprazine is shown in Figure 6. The data presented are mean duration of social investigation ± SEM of 8 rats for each group (only male offspring tested). Cariprazine administered orally increased and nearly fully reversed the social deficit induced by prenatal valproate treatment. Therefore, cariprazine was able to reduce the socio-communicational deficit induced by prenatal valproate treatment.

## Claims

1. A substance, which is trans-N-[4-[2-[4-(2,3-dichlorophenyl)piperazin-1-yl]ethyl]cyclohexyl]-N',N'-dimethylurea and/or its close analog trans-N-[4-[2-[4-(2,3-dichlorophenyl)piperazin-1-yl]ethyl]cyclohexyl]-morpholine-4 carboxamide and/or its deuterated derivatives and/or pharmaceutically acceptable salts thereof,
for use in the treatment of a core symptom of autism spectrum disorder,
wherein core symptom of autism spectrum disorder are socio-communicational deficits or restricted and repetitive behaviors; or
for use in the treatment of irritability associated with autism spectrum disorder; or
for use in the treatment of attention deficit and hyperactivity associated with autism spectrum disorder.

2. The substance for use according to claim 1, wherein the core symptoms of autism spectrum disorder are socio-communicational deficits.

3. The substance for use according to claim 1, wherein the core symptoms of autism spectrum disorder are restricted and repetitive behaviors.

4. The substance for use according to claim 1, wherein the condition to be treated is irritability associated with autism spectrum disorder.

5. The substance for use according to claim 1, wherein the condition to be treated is attention deficit and hyperactivity associated with autism spectrum disorder.

6. The substance for use according to any of claims from 1 to 5, wherein trans-N-[4-[2-[4-(2,3-dichlorophenyl)piperazin-1-yl]ethyl]cyclohexyl]-N',N'-dimethylurea is in the form of trans-N-[4-[2-[4- (2,3-dichlorophenyl)piperazin-1-yl]ethyl]cyclohexyl]-N',N'-dimethylurea hydrochloride.

7. The substance for use according to any of claims from 1 to 5, wherein trans-N-[4-[2-[4-(2,3-dichlorophenyl)piperazin-1-yl]ethyl]cyclohexyl]-morpholine-4 carboxamide is used.

8. The substance for use according to claim 7, wherein trans-N-[4-[2-[4-(2,3- dichlorophenyl)piperazin-1-yl]ethyl]cyclohexyl]-morpholine-4 carboxamide is in the form of trans-N- [4-[2-[4-(2,3-dichlorophenyl)piperazin-1-yl]ethyl]cyclohexyl]-morpholine-4 carboxamide hydrochloride.

9. The substance for use according to claim 1, wherein the autism spectrum disorder is Asperger's syndrome, atypical autism or childhood disintegrative disorder.

10. A pharmaceutical composition comprising trans-N-[4-[2-[4-(2,3-dichlorophenyl)piperazin-1- yl]ethyl]cyclohexyl]-N',N'-dimethylurea and/or its close analog trans-N-[4-[2-[4-(2,3- dichlorophenyl)piperazin-1-yl]ethyl]cyclohexyl]-morpholine-4 carboxamide and/or its deuterated derivatives and/or pharmaceutically acceptable salts thereof and a pharmaceutically acceptable excipient, for its use in the treatment of one or more core symptoms of autism spectrum disorder,
wherein core symptom of autism spectrum disorder are socio-communicational deficits or restricted and repetitive behaviors; or
for use in the treatment of irritability associated with autism spectrum disorder, or
for use in the treatment of attention deficit and hyperactivity associated with autism spectrum disorder.

11. The pharmaceutical composition for its use according to claim 10, wherein the core symptoms of autism spectrum disorder are socio-communicational deficits.

12. The pharmaceutical composition for its use according to claim 10, wherein the core symptoms of autism spectrum disorder are restricted and repetitive behaviors.

13. The pharmaceutical composition for its use according to claim 10, the condition to be treated is irritability associated with autism spectrum disorder.

14. The pharmaceutical composition for its use according to claim 10, wherein the condition to be treated is attention deficit and hyperactivity associated with autism spectrum disorder.

15. The pharmaceutical composition for its use according to claim 10, wherein the autism spectrum disorder is Asperger's syndrome, atypical autism or childhood disintegrative disorder.

## Patentansprüche

1. Substanz, die trans-N-[4-[2-[4-(2,3-Dichlorphenyl)piperazin-1-yl]ethyl]cyclohexyl]-N',N'-dimethylharnstoff und/oder dessen nahes Analogon trans-N-[4-[2-[4-(2,3-Dichlorphenyl)piperazin-1-yl]ethyl]cyclohexyl]-morpholin-4-carboxamid und/oder dessen deuterierten Derivate und/oder pharmazeutisch akzeptable Salze davon ist,
zur Verwendung bei der Behandlung eines Kernsymptoms einer Autismus-Spektrum-Störung,
wobei das Kernsymptom der Autismus-Spektrum-Störung sozio-kommunikative Defizite oder eingeschränkte und sich wiederholende Verhaltensweisen ist; oder
zur Verwendung bei der Behandlung von Reizbarkeit, die mit einer Autismus-Spektrum-Störung im Zusammenhang steht; oder
zur Verwendung bei der Behandlung von einem Aufmerksamkeitsdefizit und Hyperaktivität, die mit einer Autismus-Spektrum-Störung im Zusammenhang stehen.

2. Substanz zur Verwendung gemäß Anspruch 1, wobei die Kernsymptome der Autismus-Spektrum-Störung sozio-kommunikative Defizite sind.

3. Substanz zur Verwendung gemäß Anspruch 1, wobei die Kernsymptome der Autismus-Spektrum-Störung eingeschränkte und sich wiederholende Verhaltensweisen sind.

4. Substanz zur Verwendung gemäß Anspruch 1, wobei der zu behandelnde Zustand Reizbarkeit ist, die mit einer Autismus-Spektrum-Störung im Zusammenhang steht.

5. Substanz zur Verwendung gemäß Anspruch 1, wobei der zu behandelnde Zustand ein Aufmerksamkeitsdefizit und Hyperaktivität ist, die mit einer Autismus-Spektrum-Störung im Zusammenhang stehen.

6. Substanz zur Verwendung gemäß einem der Ansprüche 1 bis 5, wobei trans-N-[4-[2-[4-(2,3-Dichlorphenyl)piperazin-1-yl]ethyl]cyclohexyl]-N',N'-dimethylharnstoff in Form von trans-N-[4-[2-[4-(2,3-Dichlorphenyl)piperazin-1-yl]ethyl]cyclohexyl]-N',N'-dimethylharnstoff-Hydrochlorid vorliegt.

7. Substanz zur Verwendung gemäß einem der Ansprüche 1 bis 5, wobei trans-N-[4-[2-[4-(2,3-Dichlorphenyl)piperazin-1-yl]ethyl]cyclohexyl]-morpholin-4-carboxamid verwendet wird.

8. Substanz zur Verwendung gemäß Anspruch 7, wobei trans-N-[4-[2-[4-(2,3-Dichlorphenyl)piperazin-1-yl]ethyl]cyclohexyl]-morpholin-4-carboxamid in Form von trans-N-[4-[2-[4-(2,3-Dichlorphenyl)piperazin-1-yl]ethyl]cyclohexyl]-morpholin-4-carboxamid-Hydrochlorid vorliegt.

9. Substanz zur Verwendung gemäß Anspruch 1, wobei die Autismus-Spektrum-Störung das Asperger-Syndrom, atypischer Autismus oder eine desintegrative Störung in der Kindheit ist.

10. Pharmazeutische Zusammensetzung, umfassend trans-N-[4-[2-[4-(2,3-Dichlorphenyl)piperazin-1-yl]ethyl]cyclohexyl]-N',N'-dimethylharnstoff und/oder dessen nahes Analogon trans-N-[4-[2-[4-(2,3-(Dichlorphenyl)piperazin-1-yl]ethyl]cyclohexyl]-morpholin-4-carboxamid und/oder dessen deuterierte Derivate und/oder pharmazeutisch akzeptable Salze davon und einen pharmazeutisch akzeptablen Hilfsstoff, zur Verwendung bei der Behandlung eines oder mehrerer Kernsymptome einer Autismus-Spektrum-Störung,
wobei das Kernsymptom der Autismus-Spektrum-Störung sozio-kommunikative Defizite oder eingeschränkte und sich wiederholende Verhaltensweisen ist; oder
zur Verwendung bei der Behandlung von Reizbarkeit, die mit einer Autismus-Spektrum-Störung im Zusammenhang steht; oder
zur Verwendung bei der Behandlung von einem Aufmerksamkeitsdefizit und Hyperaktivität, die mit einer Autismus-Spektrum-Störung im Zusammenhang stehen.

11. Pharmazeutische Zusammensetzung zur Verwendung gemäß Anspruch 10, wobei die Kernsymptome der Autismus-Spektrum-Störung sozio-kommunikative Defizite sind.

12. Pharmazeutische Zusammensetzung zur Verwendung gemäß Anspruch 10, wobei die Kernsymptome der Autismus-Spektrum-Störung eingeschränkte und sich wiederholende Verhaltensweisen sind.

13. Pharmazeutische Zusammensetzung zur Verwendung gemäß Anspruch 10, wobei der zu behandelnde Zustand Reizbarkeit ist, die mit einer Autismus-Spektrum-Störung im Zusammenhang steht.

14. Pharmazeutische Zusammensetzung zur Verwendung gemäß Anspruch 10, wobei der zu behandelnde Zustand ein Aufmerksamkeitsdefizit und Hyperaktivität ist, die mit einer Autismus-Spektrum-Störung im Zusammenhang stehen.

15. Pharmazeutische Zusammensetzung zur Verwendung gemäß Anspruch 10, wobei die Autismus-Spektrum-Störung das Asperger-Syndrom, atypischer Autismus oder eine desintegrative Störung in der Kindheit ist.

## Revendications

1. Substance, qui est le trans-N-[4-[2-[4-(2,3-dichlorophényl)pipérazine-1-yl]éthyl]cyclohexyl]-N',N'-diméthylurée et/ou son analogue proche trans-N-[4-[2-[4-(2,3-dichlorophényl)pipérazine-1-yl]éthyl]cyclohexyl]-morpholine-4 carboxamide et/ou ses dérivés deutérés et/ou leurs sels pharmaceutiquement acceptables,
destinée à une utilisation dans le traitement d'un symptôme principal du trouble du spectre autistique,
dans laquelle le symptôme principal du trouble du spectre autistique est un déficit socio-communicationnel ou des comportements restreints et répétitifs ; ou
destinée à une utilisation dans le traitement de l'irritabilité associée au trouble du spectre autistique ; ou
destinée à une utilisation dans le traitement du déficit de l'attention et de l'hyperactivité associés au trouble du spectre autistique.

2. Substance pour utilisation selon la revendication 1, dans laquelle les symptômes principaux du trouble du spectre autistique sont des déficits socio-communicationnels.

3. Substance pour utilisation selon la revendication 1, dans laquelle les symptômes principaux du trouble du spectre autistique sont des comportements restreints et répétitifs.

4. Substance pour utilisation selon la revendication 1, dans laquelle l'affection à traiter est l'irritabilité associée au trouble du spectre autistique.

5. Substance pour utilisation selon la revendication 1, dans laquelle l'affection à traiter est le déficit de l'attention et l'hyperactivité associés aux troubles du spectre autistique.

6. Substance pour utilisation selon l'une quelconque des revendications 1 à 5, dans laquelle le trans-N-[4-[2-[4-(2,3-dichlorophényl)pipérazine-1-yl]éthyl]cyclohexyl]-N',N'-diméthylurée est sous la forme de chlorhydrate de trans-N-[4-[2-[4-(2,3-dichlorophényl)pipérazine-1-yl]éthyl]cyclohexyl]-N',N'-diméthylurée.

7. Substance pour utilisation selon l'une quelconque des revendications 1 à 5, dans laquelle le trans-N-[4-[2-[4-(2,3-dichlorophényl)pipérazine-1-yl]éthyl]cyclohexyl]-morpholine-4 carboxamide est utilisé.

8. Substance pour utilisation selon la revendication 7, dans laquelle le trans-N-[4-[2-[4-(2,3-dichlorophényl)pipérazine-1-yl]éthyl]cyclohexyl]-morpholine-4 carboxamide est sous la forme de chlorhydrate de trans-N-[4-[2-[4-(2,3-dichlorophényl)pipérazine-1-yl]éthyl]cyclohexyl]-morpholine-4 carboxamide.

9. Substance pour utilisation selon la revendication 1, dans laquelle le trouble du spectre autistique est le syndrome d'Asperger, l'autisme atypique ou le trouble désintégratif de l'enfance.

10. Composition pharmaceutique comprenant du trans-N-[4-[2-[4-(2,3-dichlorophényl)piperazin-1-yl]éthyl]cyclohexyl]-N',N'-diméthylurée et/ou son analogue proche trans-N-[4-[2-[4-(2,3-dichlorophényl)pipérazine-1-yl]éthyl]cyclohexyl]-morpholine-4 carboxamide et/ou ses dérivés deutérés et/ou leurs sels pharmaceutiquement acceptables et un excipient pharmaceutiquement acceptable, pour son utilisation dans le traitement d'un ou de plusieurs symptômes fondamentaux du trouble du spectre autistique,
dans laquelle le symptôme principal du trouble du spectre autistique est un déficit socio-communicationnel ou des comportements restreints et répétitifs ; ou
destinée à une utilisation dans le traitement de l'irritabilité associée au trouble du spectre autistique, ou
destinée à une utilisation dans le traitement du déficit de l'attention et de l'hyperactivité associés au trouble du spectre autistique.

11. Composition pharmaceutique pour utilisation selon la revendication 10, dans laquelle les symptômes principaux du trouble du spectre autistique sont des déficits socio-communicationnels.

12. Composition pharmaceutique pour utilisation selon la revendication 10, dans laquelle les symptômes principaux du trouble du spectre autistique sont des comportements restreints et répétitifs.

13. Composition pharmaceutique pour utilisation selon la revendication 10, l'affection à traiter est l'irritabilité associée au trouble du spectre autistique.

14. Composition pharmaceutique pour utilisation selon la revendication 10, dans laquelle l'affection à traiter est le déficit de l'attention et l'hyperactivité associés aux troubles du spectre autistique.

15. Composition pharmaceutique pour utilisation selon la revendication 10, dans laquelle le trouble du spectre autistique est le syndrome d'Asperger, l'autisme atypique ou le trouble désintégratif de l'enfance.
